# EUROPEAN PATENT SPECIFICATION

(11) **EP 1 702 614 B1**
(45) Date of publication and mention of the grant of the patent: **03.03.2010**
(21) Application number: 05701657.8
(22) Date of filing: 05.01.2005
(51) Int. Cl.: A61K 31/122, A61P 17/00

(54) **USE OF IDEBENONE FOR THE PREPARATION OF A TOPICALLY-APPLIED DEPIGMENTATION COMPOSITION**
VERWENDUNG VON IDEBENON ZUR HERSTELLUNG EINER TOPISCH AUFGEBRACHTEN DEPIGMENTIERUNGSZUSAMMENSETZUNG
UTILISATION DE L'IDEBENONE DANS LA PREPARATION D'UNE COMPOSITION DE DEPIGMENTATION A USAGE TOPIQUE

(30) Priority: 06.01.2004 AR 0100014
(43) Date of publication of application: 20.09.2006
(73) Proprietor: Lipotec, S.A., E-08550 Gava (ES); Creactivar S.A., Ciudad Autonoma de Buenos Aires (AR)
(72) Inventor: MARTIN LAGUENS, Rubén, Ciudad Autónoma de Buenos Aires (AR); GABRIEL ZEITUNE, Moisés, Ciudad Autónoma de Buenos Aires (AR); ELIAS MIRSON, Daniel Javier, Ciudad Autónoma de Buenos Aires (AR); HERVOY KRBAVCIC, Iván, Ciudad Autónoma de Buenos Aires (AR)
(74) Representative: Carvajal y Urquijo, Isabel
(86) International application number: PCT/ES2005/000002
(87) International publication number: WO 2005/065670

(56) References cited:
- JP-A- 11 116 470
- US-A1- 2004 197 282
- LAGUENS ET AL: "Screening of depigmenting activity of idebenone" MEDICINA CUTANEA IBERO-LATINO-AMERICANA, vol. 36, no. 4, July 2008 (2008-07), pages 183-188,

## Description

The present invention refers to a composition comprising idebenone for use in the treatment of hyperpigmentation of skin due to skin disorders, for topical application on the skin, to non-therapeutic use of idebenone in a cosmetic composition for reducing the coloration of the skin in the application site and to a pharmaceutical and/or dermatological composition comprising an effective amount of idebenone for use in the treatment of hyperpigmentation of skin due to skin disorders for topical application on the skin.

### Background of the Invention

Melanin, which is produced in cells called melanocytes, is a dark pigment found in the skin, hair, eyes and in certain nerve cells.

Melanocytes are neuroectodermal cells originating in the neural crests in the embryo which, during the fetal stage, migrate to the basal layer of the epidermis. There are also extracutaneous melanocytes distributed in other tissues.

Epidermotropic melanocytes reach the deepest layers of the epidermis in the fetal stage, being located among the basal cells at a ratio of about 1 melanocyte for every 10 basal cells, the race of the carrier being of no importance. Once in their final position, these cells emit branched extensions called dendrites, such that all basal cells are in contact with these extensions.

The primary function of melanocytes is to synthesize a dark pigment called melanin. Said pigment accumulates in the melanocyte cytoplasm in secretory granule-like ovoid structures called melanosomes which migrate through the melanocyte cytoskeleton towards the dendrites after being formed in the cell body. This process is controlled by hormones, there being hormones promoting melanin formation and others inhibiting it, as well as hormones promoting the mobilization of melanosomes towards the periphery of the dendrites and other hormones concentrating them around the nucleus. This process is extremely evident in lower animal species, although it also exists humans.

The free ends of the melanocyte dendrites are introduced in the basal keratinocyte cytoplasm, literally injecting melanosomes in the cytoplasm of these cells. Therefore, the entire epidermal basal layer and the hair follicle contain evenly distributed melanin due to the presence of melanosomes in the melanocytes on one hand, and on the other hand and to a large extent due to the incorporation of melanosomes in the basal keratinocyte, circumstantially known as melanophore. What stands out most in this process is that when the pigmented basal keratinocyte (melanophore) is divided in order to give rise to more superficial differentiated prickle cells, the melanosomes are lost and, on the other hand, the melanocyte dendrites do not inject melanosomes into differentiated prickle cells. These lost melanosomes can be housed in the intercellular space of the epidermis and then be eliminated together with ordinary desquamation of the skin. These melanosomes are completely degraded and no longer carry out their pigmenting function.

Normal human skin color is directly related to the size, configuration, type and color of the melanosomes and their distribution in melanocytes and keratinocytes. Melanin synthesis occurs exclusively in melanosomes and depends on the action of various genes.

Each melanosome is a spherical or ovoid organoid formed by a trilaminar lipoprotein cell membrane, an amorphous matrix with water, electrolytes and different solutes, in which active enzymes and a tubulin protein ultrastructure forming intramembrane tubules more or less parallel to one another are diluted.

Up until a short time ago it was believed that melanosomes were made up of only melanin and melanin-protein, a product resulting from interaction with tyrosinase. Recent studies show that melanosomes contain two different fractions, a lipid fraction with lipids located in the exterior portion of the melanosome, and a protein fraction with structural proteins forming the central portion of the melanosome. The lipid fraction is important in functional regulation of the melanosome, whereas the proteins of the matrix control its structural differentiation.

The process for forming melanosomes and their melanization can be considered to be a "cascade" of events channeled through internal regulating controls becoming involved as the melanosomes are programmed to carry out their functions. Said process can be summarized as follows:
i) Formation and organization of the melanosome components: The structural and enzymatic proteins of melanosomes are formed inside the melanosome membrane vacuoles. At an early stage, the membranes still incorporate specific proteins and lipids. The proteins formed in the rough endoplasmic reticulum, which are arranged in tubules, lamellae and filaments with no defined architectural organization, are deposited inside the vacuoles formed in the smooth endoplasmic reticulum. Microvesicles formed in the Golgi apparatus containing enzymes or post-tyrosinase regulating factors (such as dopachrome-tautomerase), which fuse with the outer membrane, are also incorporated. Tyrosinase is incorporated after its glycosylation in the Golgi complex.
   Over time, structural proteins continue to be incorporated to the melanosome, and the same occurs with tyrosinase. Structural proteins will become part of the melanosome membrane and then they pass on to the matrix.
ii) Conversion into eumelanosomes and pheomelanosomes: The pathway followed by the melanosomes at this point will depend on cysteine levels and/or the levels of compounds with sulfhydryl groups (e.g. glutathione) found inside the melanosome. If the levels are low, eumelanogenesis stimulation will occur with eumelanosome formation. In this case, the lamellae will become the predominant component of the matrix and will be arranged in parallel. Tyrosinase will be transferred from the Golgi apparatus and will be coupled to the already formed vesicles and lamellae. In the absence of an inhibiting cysteine level, tyrosinase will trigger melanin synthesis from the conversion of tyrosinase into dopaquinone. Dopaquinone will be converted into eumelanin by autoxidation processes, in addition to those processes regulated by the dopachrome conversion factor. Apparently, indole-5,6-quinone is polymerized with several of its precursors in order to form melanin inside the melanosomes. On the other hand, if cysteine levels are high, pheomelanosome will be formed and pheomelanin will be deposited on a conglomerate of not yet structured microfilaments and vesicles. Tyrosinase will convert tyrosine into dopaquinone, but the latter will spread towards the matrix and combine with cysteine, forming cysteinyldopa, subsequently modified to form pheomelanin. Cysteinyldopa competes with dopaquinone, thus changing its metabolism, without altering tyrosinase activity. Post-tyrosinase activity is then inhibited by the presence of the 5,6-hydroxyindole metabolite, which does not allow indole-5,6-quinone synthesis, decreasing the rate of melanogenesis, allowing dopaquinone to accumulate in the matrix, preventing normal eumelanin synthesis. In other words, cysteine would act as a toxic substance for melanosome, inhibiting certain enzymatic steps blocking normal conversion of intermediates into true melanin, the possibility of melanin being fixed to the melanosome structural proteins also being lost.
iii) Transfer and Degradation: The melanosomes are transferred from their synthesis site in the perikaryon to the ends of the dendrites due to the action of contractile movements of the melanocyte cytoskeleton in a melanosome selective process. Once melanosome transfer to the inner portion of the keratinocytes has begun, these latter cells phagocyte the free dendrite ends containing the melanosomes and then a membrane fusion phenomenon occurs, the melanosomes being released into the keratinocyte cytoplasm. The melanosomes are incorporated to the keratinocyte in secondary Iysosomes. There, lysosomal enzymes will begin to degrade the melanosome and its components will be diluted into the cytoplasm, possibly being reused when incorporated to a metabolic substrate pool. One of the most important features of this degradation is that melanin passes from an oxidized state to a reduced state, decreasing color intensity.

The melanosome cycle is thus completed, beginning with its synthesis in the melanocyte, being transferred to the keratinocyte and finally being degraded in a continuous process, assuring pigment distribution uniformity.

Skin melanin pigmentation can be divided into several causal components: 1) cutaneous melanin generated according to genetic programs in the absence of exposure to ultraviolet rays (constitutive skin pigmentation) and 2) immediate and delayed tanning reactions induced by direct exposure of the skin to UV radiation (facultative skin pigmentation). Facultative pigmentation changes result from a complex interaction between sunlight, hormones and tanning capacity depending on individual genetic constitution.

Constitutive pigmentation of the skin, hair and eyes is genetically determined by several genes, these genes lacking a clear dominance. Furthermore, there is a great tendency for spontaneous mutation of these genes, therefore it is not uncommon to find individuals with more than one melanocyte population, becoming mosaics for this trait.

Although melanocyte populations in human skin have regional variations, all human beings, regardless of their skin color, have about the same quantity of epidermal melanocytes in a given anatomical area. As a result, ethnic differences of skin color are mainly due to differences in melanosome properties and not to the quantity of melanocytes.

The quantity of melanosomes inside non-exposed skin melanocytes is higher in Afro-Americans, black people from Africa and in Australian Aborigines (group 2) than in white, North American European descendents and Asians (group 1). Most melanosomes are found in formation stages II and III in individuals in group 1, whereas a significant proportion of melanosomes are already completely melanized (stage IV) in individuals in group 2. Not only are the melanosomes greater in number in group 2, but they are also larger in size.

Other genetic and racial differences in constitutive pigmentation occur due to the quantity of pheomelanin in relation to eumelanin. Pheomelanin adopts a clearer, reddish color, while eumelanin is black. The different constitutive skin tones occur, therefore, due more to the concentration of one type of melanin or another than a quantitative problem.

On the other hand, it was proven by means of experiments in the 1960s that injecting alpha and beta melanotropin polypeptide hormones (or melanocyte-stimulating hormones - MSH) induced a pigmentation increase that was secondary to a melanogenesis increase inside epidermal melanocytes and an increase in transport of the melanosomes derived from the melanocytes towards the inner portion of the keratinocytes. This phenomenon is also observed when the adrenocorticotropic hormone (ACTH) is administered, since it shares or has a polypeptide sequence very similar to MSH.

It is currently accepted that the adult human pituitary gland produces significant amounts of ACTH and beta-lipotropin hormones, which are capable of melanotropic activity. These hormones would not affect melanin pigmentation in normal human beings. In fact, both said hormones and sex hormones (androgens, estrogens and progesterone) would have a very limited role in maintaining constitutive skin pigmentation in an adult human. Nevertheless, MSH is produced in the pituitary gland of a human fetus, which would affect the developing melanocyte system.

However, the best example of the role endogenous hormones would play in the darkening of constitutive skin pigmentation induced by ultraviolet radiations is melasma. This is characterized by increased regional, irregular and usually symmetrical melanization mainly in the cheek, forehead, and sometimes upper lip and neck areas.

Melasma is frequent during normal pregnancy and generally disappears gradually a short time after the pregnancy ends. Progesterones and estrogens probably lay the groundwork for this hyperpigmentation to occur, exposure to sunlight being the triggering factor and promoter of this phenomenon. In this sense, for example, melasma-like pigmentation can be observed in women using oral contraceptives. Experimental studies have further shown that administering hormones in combination with UV radiation induces a more intense cutaneous hyperpigmentation than when any of these agents is used separately. These studies have also shown that UV light in cell cultures determines increased MSH receptor activity, which suggests that a normal skin tan could be increased by melanotropins. This is supported by the less intense tan coloring obtained by people who have hypopituitarism when they are exposed to UV radiation.

Even in the absence of UV stimulation, the hormones produced during pregnancy and in certain hormone disorders are capable of increasing human melanin pigmentation. For example, in Addison's disease (suprarenal hypoadrenocorticism), high ACTH production not regulated due to the absence of corticoids promotes generalized hyperpigmentation. Pigmentation of non-exposed areas also increases during pregnancy, such as in the labia majora, the areola, the nipple, and the abdominal midline.

Melanocyte distribution is not uniform throughout the skin. In fact there are individual variations and variations within different parts of the body in a single individual. For example, in humans of all races there are over 2,000 melanocytes/mm² in the skin on the head and forearms, and generally about 1,000 melanocytes/mm² in the rest of the body. As a result, racial differences of skin pigmentation are not because of differences in the melanocyte quantity, but because of differences in melanin and melanosome synthesis.

The reasons for these regional differences in a single individual are unknown, but these differences naturally exist since birth, and even during fetal life. There is no clear correlation between usual exposure to sunlight and functional melanocytes. However, even though there is a larger quantity of melanocytes in the exposed areas than in the non-exposed areas of the forearm, an increase in the quantity of functional melanocytes in non-exposed skin after UV radiation can be observed, similar to the increase observed in skin exposed to UV light. It is not exactly known if the increase in the number of functional melanocytes is because these cells begin mitosis, or because of a recruitment of quiescent cells that become functional.

Melanocyte division is evidently important for increasing functional melanocyte production in skin irradiated with UV light. The fact that melanocytes also divide in nonirradiated skin indicates that a population turnover (although slow) is required, which could be related to the need to eliminate genetic lesions induced by intrinsic and extrinsic chemical and physical agents.

Apparent changes in skin color occur during a person's lifetime. For example, almost all black children are lighter when they are born than a week later. Freckles, which at first are only visible after sun exposure, become permanent in adolescence. The skin on the back of the hands acquires a mottled appearance in the elderly.

These quantitative changes in the epidermal melanocyte population related to the age of the individual or usual exposure to sunlight have been widely studied. A progressive age-dependent decline in melanocyte quantity can be observed in adult humans, ranging from 8 to 10% per decade of life. These are the values for non-exposed areas, since the reduction is much less in exposed areas, probably due to the stimulating effect of UV light on the melanocyte population.

In a study that correlated age with chronic sun exposure, it was found that the numerical melanocyte density in all the studied subjects was about twice that in exposed areas than in non-exposed areas but an age-related density decrease was detected in both cases. Exposed area melanocytes were more active in terms of melanin production, which explains the higher degree of pigmentation in the visible areas. Chronic sun exposure does not prevent an age-related decline in melanocyte quantity, but it does affect melanin production and induces melanocyte activation or proliferation. It is interesting to point out that keratinocytes produce mitogenic substances for melanocytes, and this production increases 6 fold if the keratinocytes are exposed to UV light.

The melanin producing unit is lost in scarring processes, so the skin of a scar is lighter than the surrounding tissue. The coloration may be recovered over time, depending on the age of the individual, sun exposure and other factors such as hormonal factors. Paradoxically there is also the contrasting case of scar hyperpigmentation, clearly indicating the complex interaction of the different elements involved in the pigmentation process.

In summary, it can be said that constitutive skin pigmentation dynamics depend on genetic and ethnic factors and on a correct interaction between keratinocytes and melanocytes, affected by an also constitutive basal hormonal state.

In contrast, facultative skin pigmentation largely depends on other external factors, such as sunlight. Sunlight acts by promoting melanocyte proliferation, melanocyte activation and melanin production, but certain cell and hormonal interaction phenomena must occur in order for it to act. Aging in turn tends to decrease normal homeostasis of the skin melanin unit, causing imbalances in pigmentation processes and pigmentation maintenance.

The brown or black color observed in pigmented cells is due to the presence of melanin. Nevertheless, this is not the only pigment that produces these colors, nor is melanin homogenous in its chemical constitution, therefore it is difficult to define what melanin pigment is. Nevertheless, melanin pigment in mammals has been divided into two main melanin types: eumelanin and pheomelanin. The first type, of a brown or black color, is insoluble, nitrogenous and derived from tyrosine. Pheomelanin, in contrast, of a yellow or red color, is soluble in alkalis, contains sulfur and is also derived from tyrosine, but through an enzymatic shunt caused by the presence of sulfur amino acids such as cysteine. Both types of melanin exist in a single individual, although eumelanin is the predominant type.

Mammal eumelanin is basically made up of indole-5,6-quinone units. The latter derives from tyrosine by the elimination of five oxygen atoms and the evolution of a carbon dioxide molecule from the tyrosine carboxylic group, being converted into dopaquinone. Indoles are derived from dopaquinone cyclization, and melanin is thought to mainly represent a poly-indole-quinone.

The precise ratios of indole subunits in melanin are probably under control of the enzymes, but they depend on precise polymerization conditions. Eumelanin is represented by a rigid chained, rod-shaped molecule formed by indole-quinone units. The physical structure of melanosomes is represented by a copolymer in which melanin and melanosome structural proteins run parallel to one another, but they may be joined at planar group sites. In elliptical melanosomes, melanin is arranged in double-helix shape, polymerized with proteins.

In contrast, from a chemical point of view pheomelanin is differentiated by the high quantity of sulfur resulting from the nucleophilic aggregate of the amino acid cysteine to the dopaquinone formed by the action of tyrosinase. Cysteine mainly interacts through a 1:6 addition to enzymatically formed dopaquinone to give the 5-S-cysteinyldopa compound. Similar intermediate compounds were also identified, depending on the pheomelanin sulfur source. Unlike dopa, cysteinyldopa is not a tyrosinase substrate.

Finally, it is likely that most melanin is a mixed type, depending on the amount of synthesized eumelanin and pheomelanin intermediaries. These compounds copolymerize to form mixed melanin, which would explain the different optical tones obtained with melanins.

In mammals, tyrosinase is an enzyme having two functions: it converts tyrosine to dopa and then converts dopa to dopaquinone, which is then cyclized and again oxidized to give rise to eumelanin formation. In contrast, if dopaquinone binds to cysteine, pheomelanin will be formed.

Tyrosinase exists in the form of three isomers, although it can be considered to be a monooxygenase containing copper and catalyzing monophenol hydroxylation and diphenol oxidation, that is, dopa (dihydroxyphenylalanine), to form a quinone. The two enzymatic activities are generally referred to as cresolase or monophenolase activity and catecholase or diphenolase activity.

There are several factors modifying the tyrosinase product, the dopachrome conversion factor being among them, which accelerates dopachrome conversion into 5,6-dihydroxyindole, the indole blocking factor inhibiting the conversion of 5,6-dihydroxyquinol into melanochrome, and the indole conversion factor accelerating the conversion of 5,6-dihydroquinol into melanochrome. Particularly the indole blocking factor and the dopachrome conversion factor are closely associated to the soluble tyrosinase isomeric forms (types I and II, those of lesser concentration inside the melanosome), whereas the dopachrome conversion factor is associated to the fixed tyrosinase isomer (type IV). These factors are auxiliary enzymatic systems among which the dopachrome-oxidoreductase (dopachrome-isomerase or dopachrome tautomerase) system stands out. Its function is to form dopachrome tautomers for forming carboxylic derivatives. Without these factors, melanin does not finish maturing to be polymerized.

There are other enzymes (peroxidase, catalase and glutamine metabolic enzymes) and metal ions acting in post-tyrosinase melanogenesis regulation in addition to dopachrome tautomerase.

In summary, melanogenesis depends on a perfect functional interaction between the tyrosinase enzyme and its substrate, tyrosine, to later give dopa and subsequently dopaquinone. The latter is taken by post-tyrosinase enzymatic factors to cause its reconversion into indole units which will end up being polymerized, to give a copolymer with melanosome structural proteins, producing eumelanin. The presence of cysteine in the melanosome matrix blocks the action of these post-tyrosinase factors, giving other intermediate soluble compounds that cannot be polymerized. However, it is common to find a mixture of partially polymerized melanins, depending on the sulfur concentration they contain. This explains the different tones found among the different melanins, since their varied structure will absorb certain wavelengths of the visible light spectrum differently according to the type of melanin in question.

This must not be confused with skin color, since it is only applicable to the pigment color. Other factors are involved in forming skin color, such as the concentration of different types of melanin, the dispersion of the latter in the melanin unit, the types of melanosomes created and the diffraction caused by the epidermis acting as a diffusing screen. The presence of other pigments in the skin are also involved in forming skin color, basically hemoglobin pigments, which are clearly seen in light skin, but are masked by melanin pigment in the epidermis in dark skin.

It frequently occurs in certain individuals that an area of the skin where the melanin density in the melanocytes is notably increased, the affected area has a skin color that is darker than the surrounding skin color. These areas are known as hyperpigmentation areas and can cause discomfort in the individual.

Cited among the most common causes of hyperpigmentation are the exaggerated response of a skin area to ultraviolet stimulation, hypersensitivity to ultraviolet light provided by exacerbating radiation action agents (such as, for example, cosmetics with bergamot oil or agents generically called phototoxins), hormonal disturbances (such as, for example, altered thyroidal hormones, and sexual, endogenous and exogenous steroids, and pregnancy), and secondary hyperpigmentation or hyperpigmentation due to or resulting from an inflammatory lesion. In particular, post-inflammatory hyperpigmentation exhibits irregular spots that are more pigmented than the surrounding skin occurring after inflammation due to a skin lesion resulting from a condition such as acne, folliculitis, eczema, hair removal, scratching, etc. Said post-inflammatory hyperpigmentation is resolved slowly but may persist for months and even years, and it is frequently the reason for medical visits, many times requiring professional care.

To date, several cutaneous topical compositions containing one or more ingredients capable of reducing melanin density in cutaneous melanocytes have been disclosed. Such ingredients are generically referred to as depigmenting agents or bleaching agents. Said agents are generally absorbed through the lower layers of the skin, inhibiting melanin formation in melanocytes and specifically acting on certain stages of melanogenesis. The most frequent depigmenting agents are based on hydroquinone or derivatives thereof, such as benzyl-oxy-phenol and hydroquinone monobenzylether (US patent 3,060,097). This last compound has the drawback that it is not suitably metabolized when absorbed through the skin, for which it is associated with irreversible depigmentation events simulating vitiligo (gradually spreading cutaneous depigmentation areas, often with a hyperpigmented edge). Benzyl-oxy-phenol also has the drawback that it is transported by the lymphatic system to other areas of the skin, far from the application site, where it may also exercise a lightening effect.

Also proposed as a cutaneous depigmenting agent is the compound methoxyphenol, a hydroquinone ether, which has been used in pharmaceutical depigmenting compositions, but it has the drawback that, as it is relatively insoluble in aqueous media, it is difficult to be suitably incorporated in cosmetic or dermatological formulations.

Other compounds used to depigment the skin are 4-isopropyl catechol, a substituted hydroquinone derivative (South African patent application 716,890) and hydroquinone fatty acid mono and di-esters (European patent application number 82301102.8)

The direct use of hydroquinone in cosmetics for treating hyperpigmentation has also been proposed since it is effective, soluble in water and quickly metabolized and excreted. Nevertheless, hydroquinone has the drawback that it is unstable in alkaline medium and is oxidized to the quinine form, which gives a brownish color to any pharmaceutical composition containing it. It is necessary to incorporate an antioxidant to the composition, such as ascorbic acid, to prevent this oxidization. In fact, stabilization of the hydroquinone molecule, for example, has been proposed upon incorporating it to an anhydrous medium. In this sense, US patent 4,466,955 discloses a cosmetic preparation in which hydroquinone is dissolved in fatty esters, and the resulting solution is incorporated to a cosmetic, non-aqueous cream base in which hydroquinone is more stable and less prone to oxidization. This is because oxygen is less soluble in waxes than in water and, therefore, the oxidization process occurs to a lesser extent. Furthermore, according to that disclosed, this preparation favors cutaneous absorption of hydroquinone.

Hydroquinone is the generic name for the compound 1,4 benzenediol or p-dihydroxybenzene, which has a molecular weight of 110.0. Its action mechanism is prone to inhibit enzymatic tyrosine oxidization to 3,4-dihydroxyphenyl-alanine (DOPA) and for suppressing other melanocyte metabolic processes.

The main drawback of hydroquinone is that it is also a skin irritant, possibly causing paradoxical hyperpigmentation called ochronosis. The carcinogenetic potency of hydroquinone has also recently been disclosed, as at least 5 cases of cutaneous melanomas have been reported in a group of workers in daily contact with this substance.

Idebenone, on the other hand, is a benzoquinone with pharmacodynamic properties that have been established in drugs with cytoprotective effects, as disclosed in US patent 4,271,083 for example. Idebenone is the generic name of the compound 6-(10-hydroxydecyl)-2,3-dimethoxy-5-methyl-1,4-benzoquinone (JP-B-62 3134 (1987), US patent number 4,139,545). Data obtained from *in vitro* assays suggest that the cytoprotective action of idebenone is reached by facilitating convergence of electrons in the mitochondrial respiratory cycle, inhibiting lipid peroxidation, reducing non-respiratory oxygen consumption and stimulating ATP formulation.

Idebenone is considered a synthetic Q10 Coenzyme and is used, administered orally, to improve cognitive disorders, Alzheimer's disease, dementia and cerebral vascular disorders, and as a cardiac cytoprotective agent. Due to its antioxidant properties, it is administered orally either alone or in combination with other active ingredients which also preferably have antioxidant properties, such as vitamin E for example.

Patent documents DE 3,049,039, EP 0,788,793, US 4,436,753, US 5,059,627 and US 5,916,925 disclose oral, parenteral or percutaneous preparations comprising idebenone or its derivatives that can be used in treating dementia, blood circulation disturbances or to induce neural growth factors. Particularly JP patent JP 1,279,818 discloses the use of idebenone and its derivatives in different preparations that can be used to provide exogenous color to hair (idebenone is a powder having a strong orangish color). To date no significant toxic effects have been reported for idebenone (Arzneim. Forsch/drug res. 35 (II), 11, pp. 1704, 1985).

It has surprisingly been discovered now that a preparation for topical cutaneous use comprising idebenone can cause a significant reduction of pigment concentration in pigmented areas without causing significant side effects.

### DETAILED DESCRIPTION OF THE INVENTION

This invention is defined in the claims.

This invention is directed to a composition comprising idebenone for use in the treatment of hyperpigmentation of skin due to skin disorders, for topical application on the skin, to non-therapeutic use of idebenone in a cosmetic composition for reducing the coloration of the skin in the application site and to a pharmaceutical and/or dermatological composition comprising an effective amount of idebenone for use in the treatment of hyperpigmentation of skin due to skin disorders for topical application on the skin.

In addition, in this document the term "reduce skin coloration" must be understood as reducing the skin tone until obtaining a colorimetric scale reduction visible to the naked eye.

The composition for use according to the invention intended for topical application on the skin can be found, for example, in the form of cream, gel, an occlusive patch, emulsion or aerosol. The composition is preferably found in cream form (O/W). Also according to the invention, idebenone could be compr sed in a controlled release topical application, particularly in which idebenone is liposomal or complex-forming. The formulation of this type of compositions is within the s ate of the art.

The composition intended for being applied on the skin preferably comprises idebenone or a derivative thereof in an amount comprised between 0.1% and 10% w/w. Even more preferably, idebenone or its derivative is in an amount comprised between 0.3% and 5% w/w.

Examples of derivative compounds of idebenone may be, among others, those disclosed in patent documents US 4,139,545, US 4,436,753, DE 3,049,039, EP 0,788,793, US 4,436,753, US 5,059,627 and US 5,916,925.

The composition for use according to the invention could be formulated by a person skilled in the art, using known cosmetically or pharmaceutically acceptable excipients. The composition preferably comprises oily and hydrosoluble components. Examples of oily components are autoemulsifiable wax, vaseline, isopropyl myristate and cetyl alcohol. Examples of hydrosoluble components are glycerin, methylparaben and propylparaben. The composition could also contain beneficial agents for the skin such as moisturizing agents, hydrating agents and vitamins, which are known and can be chosen by a person skilled in the art. If required, the composition may further comprise cosmetically and/or pharmaceutically acceptable antioxidants and sun filters and/or screens.

### EXAMPLES

### EXAMPLE 1

### i) Aqueous cream formulations (O/W) containing idebenone (IDB)

**Table 1:**

| **INGREDIENTS** | **% BY WEIGHT** | | | | | |
|---|---|---|---|---|---|---|
| Water | Q.s. 100 g of cream | | | | | |
| Non-ionic autoemulsifiable wax | 6.00 | 6.00 | 6.00 | 6.00 | 6.00 | 6.00 |
| Vaseline | 5.00 | 5.00 | 5.00 | 5.00 | 5.00 | 5.00 |
| Glycerin | 5.00 | 5.00 | 5.00 | 5.00 | 5.00 | 5.00 |
| Isopropyl myristate | 2.00 | 2.00 | 2.00 | 2.00 | 2.00 | 2.00 |
| Cetyl alcohol | 1.50 | 1.50 | 1.50 | 1.50 | 1.50 | 1.50 |
| Methylparaben (Nipagin) | 0.20 | 0.20 | 0.20 | 0.20 | 0.20 | 0.20 |
| Propyparaben (Nipasol) | 0.10 | 0.10 | 0.10 | 0.10 | 0.10 | 0.10 |
| Idebenone | 0.10 | 0.20 | 0.3 | 0.5 | 2.50 | 4 |

### ii) Process for preparing creams containing idebenone.

The oily components (A) of the formulation (autoemulsifiable wax, vaseline, isopropyl myristate and cetyl alcohol) are melted and heated up to a temperature of 70/75°C. The hydrosoluble components (B) of the formulation (glycerin, methylparaben, and propylparaben) are dissolved in the required amount of water, as described above, and they are heated at 70/75°C.

The oily phase (A) is then added to the aqueous phase (B) while vigorously and constantly stirring, controlling the temperature of the components. It is cooled in a water bath and slowly stirred until the temperature reaches 45°C. Once the desired temperature has been reached, the required amount of idebenone is dissolved, as described above, in 0.5 ml of ethanol and the previous preparation is added while slowly stirring until obtaining a homogenous preparation. It is allowed to cool to the desired consistency anc suitably packaged.

In cases in whic - idebenone concentration exceeds 3%, it may be necessary to increase the proportion of components in the oily phase o achieve solubilization of the active ingredient. If the required idebenone concentration far exceeds 4%, the base aqueous cream my be replaced with an oily cream (W/O)

### EXAMPLE 2

### Effects of applying a composition comprising idebenone on the skin of test animals

Creams containing 5% and 2.5% w/w idebenone (IDB) in a neutral phase were prepared. Said creams and a cream having an identical formulation but without idebenone were applied on nude adult mice with 3 mor ths of age- The cream with idebenone was applied on the right half of the body of the animals and the control cream on the left half according to the scheme detailed below. The animals were sacrificed 1, 2, 3 or 4 hours after applying the cream (p.a.)
Batch A: animal treated with 5% IDB (1 hour p.a.)
Batch B: animal treated with 2.5% IDB (1 hour p.a.)
Batch C: animal treated with 5% IDB (2 hours p.a.)
Batch D: animal treated with 2.5% IDB (2 hours p.a.)
Batch E: animal treated with 5% IDB (3 hours p.a.)
Batch F: animal treated with 2.5% IDB (3 hours p.a.)
Batch G: animal treated with 5% IDB (4 hours p.a.)
Batch H: animal treated with 2.5% IDB (4 hours p.a.)

The treated and control skin areas were removed and divided into 3 sections: one for dosing idebenone by means of gas chromatography, one for determining moisture retention and the third one for paraffin inclusion.

*Idebenone dosing by gas chromatography*: The treated and control skin sections were placed epidermis side down on a cryosta: stage and 300 micron thick sections parallel to the surface were obtained in such a manner so as to obtain a deep cut, a surface cut and a medium cut. Each cut was identified, homogenized and resuspended in acetone. Each extract was analyzed by gas chromatography using a Hewlett Packard 5890 gas chromatograph, using a capillary column with methyl silicone as stationary phase (HP-IMS, 25m x 0.2 nm, 0.33µ thick film). The temperature program consisted of an initial temperature of 100°C for 3 minutes to then continue heating with 20° temperature increments until reaching 300°C. This last temperature was maintained for 5 minutes. A helium flow of 0.7 ml/min was used. The chromatograph was provided with a quadrupole mass detector coupled thereto (HP model 5972) and electronic impact ionization at 70 eV was used in SCAN mode with a mass scanning mode of 50 to 600 m/z. 2 µl of sample were injected in 1/25 split mode with an injector temperature of 250°C and an interface temperature of 280°C.

*Moisture determination*: Each treated and control skin section was weighed on an analytical balance and then placed in an oven at 120°C for 30 minutes. The samples were then cooled for 30 minutes at room ten perature to then be weighed again. The moisture percentage was calculated according to the following formula:

### PRE-EVAPORATION WEIGHT - POST-EVAPORATION WEIGHT

### PRIOR WEIGHT

The increase in the treated skin water content ir relation to the control skin water content was thus calculated by dividing the percentage obtained in each treated section by the percentage obtained in the respective control section.

*Morphological and morphometric studies*: Thickness of the epidermis, the horny layer, the papillary dermis and reticular dermis was measured in at least 10 different points, the result being expressed as the average of the 10 measurements. Capillary vessel diameter was also measured in at least 10 points, expressing them by means of their average. The measurements were carried out using a Quantimet 500+ (Leica) image processing equipment. Special dyes for connective tissue and Giemsa, methylene blue, toluidine blue (to observe metachromasia and interstitial water distribution) and Schorr stains (to observe keratin behavior) were prepared.

*Immunohistochemical studies*: Heat shock protein (HSP) overexpression was determined in alternating cuts of the material included in paraffin by means of enzymatic immunolabeling, using monoclonal antibodies against HSP27 (Dako Labs) and developed with an APAAP kit (Dako Labs).

*Morphology*: The skin of the animals treated with 5% and 2.5% IDB cream did not show morphological alterations, the microscopic images being similar to those of the control skins. Special staining techniques did not show differences between the different samples examined, even in the different treatment times. Nor were there modifications in the stains with Schorr staining for observing the different qualities of keratins.

*Epidermal morphometry*: The results are summarized in Table II. It can be seen that no significant differences were detected in the thickness of the epidermis or of the horny layer between the treated skin and control skin. Nor were significant differences detected neither in the two tested IDB concentrations nor in the different cream application times. Therefore, it can be concluded that top cally applied idebenone does not substantially modify epidermal thickness, thus exhibiting local innocuousness.

**Table II: Total epidermis and horny layer thickness in the skin of mice treated with creams containing 5 %and 2.5% idebenone and their comparison with skin treated with a neutral cream without idebenone as a control. The values are expressed in microns, as an average of 10 measurements performed (figures rounded to two decimals). In the case the controls, the values are expressed as the average of the total measurements, that is, two animals per point, since two controls were used, one for the cream containing 5% IDB and another one for the :ream containing 2.5% IDB.**

| | | **1 hour p.a.** | **2 hours p.a.** | **3 hours p.a.** | **4 hours p.a.** |
|---|---|---|---|---|---|
| **5% IDB** | Total epidermis | 60.37 | 58.44 | 60.04 | 59.73 |
| | Horny layer | 32.40 | 32.23 | 33.25 | 32.09 |
| **2.5% IDB** | Total epidermis | 59.73 | 60.21 | 58.48 | 57.47 |
| | Horny layer | 33.28 | 32.79 | 31.02 | 32.43 |
| **Control** | Total epidermis | 58.92 | 59.65 | 57.87 | 60.11 |
| | Horny layer | 30.96 | 31.37 | 32.03 | 31.55 |

*Dermal morphometry*: Thicknesses of the papillary dermis and the reticular dermis did not significantly vary between the different an mals examined. Therefore, it can be concluded that topically applied idebenone does not substantially modify epidermal thicknesses, thus showing local innocuousness

*Vascular diameters*: The results are summarized it Table III. It can be seen that there are no significant differences in microcirculation vascular diameters between the skins treated with IDB ard the control skins, nor are there any significant differences in the tested IDB concentrations or in the different cream application times. Therefore, it can be concluded that topically applied idebenone does not substantially modify microcirculation vascular diameters, thus showing the lack of local hemodynamic modifications.

**Table III: Dermal capillary vessel diameters of skins treated with a cream containing 5% IDB, a cream containing 2.5% IDB and base cream (without IDB). Diameters are expressed in microns as an average of ¹en measurements performed (figures rounded to two decimals). In the case of the controls, values are expressed as the average of the total measurements, that is, two animals per point since two controls were used, one for the cream containing 5% IDB and another one for the cream containing 2.5% IDB.**

| | **1 hour** | **2 hours** | **3 hours** | **4 hours** |
|---|---|---|---|---|
| | **p.a.** | **p.a.** | **p.a.** | **p.a.** |
| 5% IDB | 6.70 | 8.77 | 5.88 | 7.03 |
| 2.5% IDB | 6.55 | 7.03 | 6.32 | 6.76 |
| Control | 7.00 | 7.09 | 6.84 | 6.91 |

*HSP overexpression*: No differences were detected in HSP expression between the epidermises treated with the control cream (without IDB) and the epidermises treated with the cream containing IDB. Nor were there differences between the skins treated at different post-application times with the creams containing 5% and 2.5% IDB. Therefore, it can be concluded that treatment with IDB at the concentrations studied dos not increase epidermal HSP expression in mice skin, so it can be inferred that there is no epidermal injury justifying overexpression of this cellular defense system against aggression.

*Moisture determination*: The weights of the treated and control skins before and after evaporating treatment show a similar moisture percentage in the groups treated with IDB and the control groups. Nor were significant differences detected between the different post-application times. The results are summarized in Table IV.

**Table IV: Moisture percentages and water retention capacity in treated skin with respect to the controls. Values are expressed in grams as the average of the measurements performed at 1, 2, 3 and 4 hours after applying the creams.**

| | **Previous weight** | **Post weight** | **Difference** | **Percentage** | **Retention** |
|---|---|---|---|---|---|
| **5% IDB** | 1.3231 | 1.0391 | 0.2840 | 21.46% | 0.89 times |
| **2.5% IDB** | 0.9904 | 0.7616 | 0.2288 | 23.10% | 0.96 times |
| **Control** | 1.0924 | 0.8300 | 0.2624 | 24.02% | |

Therefore, it can be concluded that treatment with IDB at the studied concentrations does not modify the amount of water in tissues (the difference in water retention between the skins treated with IDB and the ski is treated with cream without IDB is not statistically significant).

*Penetration of ID3 in the skin:* Two hours after it was applied, an IDB peak at a retention time of 17.6 minutes in the most superficial cut (this cut includes the entire epidermis and the top portion of the dermis) was obtained, both in the sample treated with 5% IDB and with the sample treated with 2.5% IDB. In contrast, the samples corresponding to deep and mid sections (reticular dermis and hypodermis) did not show this peak. On the other hand, none of the samples showed this peak (neither those treated with IDB, or the control samples) 1 hour a ter the application. Therefore, it can be concluded that IDB penetrates the skin and is retained up to two hours after its application in the most superficial sections thereof.

### EXAMPLE 3

### Effects of applying compositions comprising different idebenone concentrations on human skin.

Skin samples from patients identified as patients 1, 2 and 3, were used for these experiments, and the following procedures were performed on them:

### Patients 1 and 2:

Breast skin was used in two patients (both women, aged 74 and 71, respectively) who would undergo a radical mastectomy and a simple mastectomy due to the presence of breast carcinoma and florid dysplasia.

The breast surface was divided into three similar surface territories 45 minutes before being taken to the operating room, and about 12C mg of cream containing 5% IDB were added in the first of these territories by means of gentle circular massaging until the cream was completely absorbed. The two remaining sections were treated similarly, using cream containing about 120 mg of cream containing 2.5% IDB and control cream. Once in the operating room, and prior to preparing the operating field, the breast surface was washed with 95° ethanol in order to remove possible excess cream.

Immediately after the operation (average surgical activity time: 45 minutes), about 4 cm² of a skin section from each area was resected, identifying it correctly. Once the section was resected, the subcutaneous adipose tissue was carefully removed so as to obtaining only epidermis and dermis.

### Patient 3:

The breast skin of a patient (female, 54 years of age) who was going to undergo a radical mastectomy due to the presence of a breast carcinoma was used.

The breast surface was divided into three similar surface territories 45 minutes before being taken to the operating room, and about 120 mg of cream containing 5% IDB were added in the first of these territories by means of gentle circular massaging until the cream was completely absorbed. The two remaining sections were treated similarly, using cream containing about 120 mg of cream containing 0.5% IDB and control cream. Once in the operating room, and prior to preparing the operating field, the breast surface was washed with 95° ethanol in order to remove possible excess cream.

Immediately after the operation (average surgical activity time: 45 minutes), about 4 cm² of a skin section from each area was resected, identifying it correctly. Once the section was resected, the subcutaneous adipose tissue was carefully removed so as to obtaining only epidermis and dermis.

The effect of applying creams containing 0.5%, 2.5% and 5% of idebenone on the morphology and morphometry of the different cu:aneous layers, of the blood vessels of the dermal plexus, and on HSP expression was studied on the skin samples of patients 1, 2 and 3 as manner of evaluating the epidermal response to a possible deleterious effect of the creams.

The skin sections treated with creams containing IDB and with control cream were fixed in formol solution, were included in paraffin and colored with histological staining techniques by means of hematoxylin and eosin, Schorr, Giemsa, and toluidine blue.

*Morphometry*: The epidermal thickness, horny layer, papillary dermis, reticular dermis and dermal plexus capillary diameters were measured in the histological cuts.

*Immunohistochemistry*: Alternating cuts included in paraffin were dewaxed and incubated with a commercial antiserum against HSP27 (Dako Labs). The reaction was developed using an APAAP kit (Dako).

*Macroscopic appearance*: The areas of skin treated with creams containing IDB, with control creams without IDB and untreated skins showed no significant differences when they were observed by the naked eye. T herefore, it can be concluded that treatment with creams containing IDB at the different tested concentrations did not cause irritation during the time they were applied.

*Microscopy*: No structural observations were detected in the skins treated with the control creams or in the skins treated with creams containing different IDB concentrations, even when special staining techniques were used. In particular, no accumulation of interstitial fluid, no vascular alterations and no accumulation of inflammatory elements in the dermis were detected. All studied epidermal layers maintained their integrity and architecture.

*Morphometric analysis of epidermal thicknesses*: The following table briefly summarizes the obtained results.

**Table V: Total epidermis and horny layer thickness values in breast skin from volunteers treated with creams containing IDB at different concentrations and control creams for different time periods. The obtained values are expressed in microns as a result of the average of 10 measurements that were perfo med.**

| | **Epidermal thickness** | **Horny layer thickness** |
|---|---|---|
| | | |

| **Patient 1** | | |
|---|---|---|
| **5% IDB** | 62.40 | 35.80 |
| **2.5% IDB** | 61.31 | 32.89 |
| **Control** | 61.40 | 36.32 |
| | | |

| **Patient 2** | | |
|---|---|---|
| **5% IDB** | 72.81 | 23.20 |
| **2.5% IDB** | 64.82 | 27.03 |
| **Control** | 72.31 | 31.02 |
| | | |

| **Patient 3** | | |
|---|---|---|
| **5% IDB** | 49.01 | 21.97 |
| **0.5% IDB** | 49.06 | 23.90 |
| **Control** | 48.95 | 25.87 |

Therefore it can be concluded that application on the skin of creams containing different IDB concentrations does not significantly modify epidermal thicknesses.

*Morphometry of the dermis*: No significant differences were detected in the measurements of reticular dermis and papillary dern is thicknesses between the different evaluated samples. Therefore, it can be concluded that the application of creams containing different IDB concentrations does not cause immediate dermal modifications.

*Morphometry of vessels in the dermal plexus*: No significant differences were detected in the measurements of vascular diameters in the different evaluated samples. The obtained results are summarized in the following table:

**Table VI: Vascular diameters of vessels in the dermal plexus in skins treated with creams containing different IDB concentrations and control cream. The obtained values are expressed n microns as a result of the average of 10 measurements performed.**

| **Vascular diameters** | |
|---|---|
| | |

| ***Patient 1*** | |
|---|---|
| 5% IDB | 12.42 |
| 2.5% IDB | 9.15 |
| Control | 11.42 |
| | |

| ***Patient 2*** | |
|---|---|
| 5% IDB | 11.90 |
| 2.5% IDB | 12.30 |
| Control | 11.82 |
| | |

| ***Patient 3*** | |
|---|---|
| 5% IDB | 7.71 |
| 0.5% IDB | 7.92 |
| Control | 7.85 |

From the obtained results, it can be concluded that the application of creams containing different IDB concentrations does not modify the vascular tone of treated skin.

*HSP27 distribution and expression:* No differences were detected in HSP expression and distribution in the different samples treated with creams containing different IDB concentrations and with control creams (without IDB) in any of the evaluated patients. From the obtained results, it can be concluded that the application of creams containing different IDB concentrations does not modify HSP27 expression and distribution, this protein being a parameter of the epidermal response to a possible injury.

### EXAMPLE 4

### Effects of applying a composition comprising idebenone applied on the skin of human beings:

### Moisture determination

Breast skin of the patient identified as Patient 3 in the previous example was used.

A base cream such as the one used in Example 1 was fractioned into 3 aliquots, adding a sufficient amount of idebenone (99.3% pure) being added so as to obtain a final IDB concentration of 5%. A sufficient amount of idebenone (99.3% pure) was added to the second aliquot so as to obtain a final ID3 concentration of 0.5%, and the third aliquot was used as a control (with no IDB aggregate).

*Moisture determination*: Each treated and control skin section was weighed on an analytical balance and then placed in an oven at 120°C for 30 minutes. The samples were then coo ed for 30 minutes at room temperature to then be weighed again. The moisture percentage was calculated according to the following formula:

### PRE-EVAPORATION WEIGHT - POST-EVAPORATION WEIGHT

### PRIOR WEIGHT

The increase of water in the treated skin in relation to the control skin was thus calculated, dividing the percentage obtained in each treated section by the percentage obtained in the respective control. The obtained results are briefly summarized in the following table:

**Table VII**

| | **Previous weight** | **Post weight.** | **Difference** | **Percentage** | **Retention** |
|---|---|---|---|---|---|
| **5% IDB** | 3.6308 | 2.6706 | 0.9602 | 26.45% | 0.84 times |
| **0.5% IDB** | 2.2653 | 1.5846 | 0.6807 | 30.04% | 0.95 times |
| **Control** | 2.1288 | 1.4560 | 0.6728 | 31.60% | |

Therefore, IDB applied topically on the skin at the studied concentrations did not increase the amount of water contained in the skin.

### EXAMPLE 5

### Melanogenesis inhibition by idebenone.

*In vitro* determinations were performed following the method devised by Dooley et al. (Skin Pharmacol. 994;7:188-200) in order to evaluate the capacity of idebenone to inhibit melanogenesis, according to the process described below:

### Materials and Methods:

*Cells*: The study was carried out using a cell line derived from human melanoma, provided by ABAC (Asociación Banco Argentino de Células- *Cell Bank of Argentina*), with melanin synthesis capability (SK-MEL-28, ATCC origin). The cells were grown in plastic culture bottles or in 24-well plastic dishes in a modified Eagle medium (DMEM) supplemented with 10% fetal bovine serum (FBS) and were maintained at 37°C in a 5% CO₂ atmosphere.

*Assayed compounds*: Idebenone, provided by Drogueria Saporiti, Argentina, batch 02107, with 99.80% purity calculated according to the dry drug. Hydroquinone (1,4- benzenediol), provided by Drogueria Saporiti, Argentina, batch 010715, with 99.85% purity calculated according to the dry drug.

*Study of in vitro compounds* : SK-MEL cells detached with trypsin were seeded on plastic 24-well dishes (density of 1 x 105 cells per wel) and incubated for 24 hours in modified Eagle medium (DMEM) supplemented with 10% fetal bovine serum (FBS) prior to treating with the compound to be evaluated. After 24 hours have lapsed, the medium was replaced with 990 µl of fresh medium. 10 µl of sterile vehicle (50% propylene glycol, 30% ethanol and 20% distilled water) containing different concentrations of the compounds to be evaluated were added to this fresh medium. The SK-MEL cells tolerated the 1% vehicle well (final concentration: 0.5% propylene glycol and 0.3% ethanol).

Decimal dilutions of each compound in the sterile vehicle were prepared in order to obtain final concentrations of the compound to be studied ranging between 1.000 and 0.01 µg/ml. This process was repeated daily for three days.

On the fourth day, the cells were not treated, and the remaining adherent cells were assayed on the fifth day according to the methods described below. The cells were thus continuously exposed to the compounds under study for the 5 days the culture lasted. All the concentrations of the compounds were studied in triplicate, comparing the mean of the 3 wells treated with the compounds with those treated with the vehicle alone.

*Melanin content determination*: The SK-MEL cell melanin content was determined after stirring the culture medium and washing the cells with PBS. The cells were then Iysated by means of adding 1 ml of 1 N NaOH to each well and repeated manual pipetting. The raw cell extract was analyzed usir,g a spectrophotometer at 400 and 475 nm to determine the melanin and dopaquincne content, respectively. The results were expressed as a percentage of the cell cultures treated with the vehicle.

*Cell number quantitation with violet crystal*: A sta n with a violet crystal aqueous solution was used for the purpose of determining the number of cells adhering to the plastic surviving *in vitro* treatment by means of indirect methods. After the treatment period, the medium was decanted from the wells by turning the dish over and was replaced with 0.5 ml of 0.1% violet crystal (in 10% ethanol) per well. The dishes were stained for 5 minutes at room temperature on a rotary platform, with gentle stirring. Then the excess colorant was decanted by turning the dish over and the entire dish was submerged 4 times in distilled water in a suitable container. After rinsing, the dishes were turned over and allowed to drip on absorbent paper until the excess water was completely removed. The violet crystal retained in :he adherent cells was later extracted by means of adding 1 ml of ethanol (95%) per well. The plates were finally placed on a rotary agitator for 30 minutes at room temperature. The optical densities of the alcohol samples were measured with the aid of a spectrophotometer (wavelength= 590 nm) using plastic cuvettes with 95% ethanol as a bank. When the cell densities were very high (for example, when optical densities exceeded 2.0), dilutions of each sample in 95% ethanol were prepared and were thus spectrophotometrically analyzed.

In order to determine the fraction of cells surviving treatment at a specific concentration of the experimental agent, the OD₅₉₀ of the treated wells (average of three measurements) was divided by the OD₅₉₀ of the wells used with a control vehicle (average of three measurements). The cell survival percentage (expressed as a percentage of the control vehicle) was obtained by multiplying said fraction by 100.

*Measuring melanin content*: Table VIII shows the average OD₄₀₀ and OD₄₇₅ values of three simultaneous measurements for idebenor e and hydroquinone dilutions.

**Table VIII**

| **IDEBENONE** | | |
|---|---|---|
| **Dilution** | OD₄₀₀ | OD₄₇₅ |
| **-1** | 0.0156 | 0.0011 |
| **-2** | 0.0140 | 0.0016 |
| **-3** | 0.0123 | 0.0043 |
| **-4** | 0.0096 | 0.0053 |
| **-5** | 0.0066 | 0.0040 |
| **Control** | 0.0236 | 0.0116 |

| **HYDROQUINONE** | | |
|---|---|---|
| **Dilution** | OD₄₀₀ | OD₄₇₅ |
| **-1** | 0.007 | 0.0001 |
| **-2** | 0.012 | 0.0013 |
| **-3** | 0.021 | 0.0083 |
| **-4** | 0.036 | 0.0163 |
| **-5** | 0.034 | 0.0163 |
| **Control** | 0.033 | 0.0163 |

and, expressed as percentages:

| I**DEBENONE** | | |
|---|---|---|
| **Dilution** | OD₄₀₀ | OD₄₇₅ |
| **-1** | 28% | 9% |
| **-2** | 40% | 14% |
| **-3** | 52% | 37% |
| **-4** | 59% | 46% |
| **-5** | 66% | 35% |
| **Control** | 100% | 100% |

| **HYDROQUINONE** | | |
|---|---|---|
| **Dilution** | OD₄₀₀ | OD₄₇₅ |
| **-1** | 21% | 0% |
| **-2** | 36% | 8% |
| **-3** | 64% | 51% |
| **-4** | 109% | 100% |
| **-5** | 103% | 100% |
| **Control** | 100% | 100% |

it can be concluded based on the obtained results that in the model used, the idebenone compound inhibits melanin synthesis in a dose-dependent manner. Although at high concentrations the effect of idebenone could be considered similar to that of hydroquinone, after certain dilution, the latter stops being effective. In contrast, the idebenone compound continues inhibiting even at greater dilutions.

Dopaquinone production is also affected in a manner similar to melanin synthesis, indicating that the inhibition is complex, inh bition possibly occurring at several sites of the melanin metabolic pathway.

*Cell number quantitation with violet crystal*: The following table summarizes the percentages of viable cells after treatment with different idebenone and hydroquinone dilutions.

**Table IX**

| **IDEBENONE** | |
|---|---|
| **Dilution** | Percentage |
| **-1** | 52% |
| **-2** | 82% |
| **-3** | 98% |
| **-4** | 100% |
| **-5** | 100% |
| **Control** | 100% |

| **HYDROQUINONE** | |
|---|---|
| **Dilution** | Percentage |
| **-1** | 34% |
| **-2** | 58% |
| **-3** | 77% |
| **-4** | 89% |
| **-5** | 95% |
| **Control** | 100% |

It can be concluded based on the obtained results that high idebenone concentrations may be toxic for cells. Something similar also occurs with hydroquinone, although toxicity for hydroquinone was higher. Given that this toxicity could interfere with reading optical melanin and dopaquinone densit es, similar experiments were carried out using other dilutions, different incubation times and using only idebenone (since the effects of hydroquinone on this system are already disclosed in the literature). These are described in the following examples.

### EXAMPLE 6

### Melanogenesis inhibition after 1 day of treatment with idebenone

An experiment similar to the one described in the previous example was designed for the purpose of establishing whether idebenone inhibits melanogenesis in cells in culture, but in this case the cells were treated or only 1 day, whereas the results were evaluated on the third day. The presence or absence of cytopathic effects on the cell cultures was also evaluated by means of using an inverted microscope, beginning with a -2 dilution and reaching -6 dilution.

Table X shows 400 and 475 optical densities (for melanin and dopaquinone, respectively) of the different assayed dilutions, and their respective percentages.

**Table X**

| **Dilution** | **OD₄₀₀** | **Percentage** | **OD₄₇₅** | **Percentage** |
|---|---|---|---|---|
| **-2** | 0.0040 | 14.65% | 0.0030 | 18.75% |
| **-3** | 0.0133 | 48.72% | 0.0050 | 31.25% |
| **-4** | 0.0243 | 89.01% | 0.0123 | 76.87% |
| **-5** | 0.0343 | 125.64% | 0.0196 | 122.5% |
| **-6** | 0.0313 | 114.65% | 0.0200 | 125.0% |
| **Control** | 0.0273 | 100% | 0.0160 | 100% |

It can be inferred from these results that idebenone begins to inhibit both melanin synthesis and synthesis of its by-products (for example dopaquinone) 24 hours after treatment in a dose-dependent manner. The -1 dilution was not taken into account in this experiment since the cultures had cytopathic effects. The remaining dilutions showed no cytopathic effect whatsoever, as a result of which it can be considered that idebenone is not cytotoxic at the doses used. It can be highlighted that the -5 and -6 dilutions had a higher amount of melanin and dopaquinone than the controls. This could simply be due to an error in the method and therefore not be significant. However, without being limited by a particular theory, it would be possible that with the incubation time used and at these dilutions, the idebenone compound may exercise a melanogenesis stimulation effect which is probably temporary.

### EXAMPLE 7

An experiment similar to the one described n the previous example was designed for the purpose of establishing whether idebenone inhibits melanogenesis in cells in culture, but in this case the cells were treated for 2 days and the results were evaluated on the fourth day. The presence or absence of cytopathic effects on the cell cultures was also evaluated by means of using an inverted microscope, evaluating only the --4, -5 and -6 dilutions.

Table XI shows 400 and 475 optical densities (for melanin and dopaquinone, respectively) of the different assayed dilutions and their respective percentages.

**Table XI**

| **Dilution** | **OD₄₀₀** | | **Percentage** | **OD₄₇₅** | **Percentage** |
|---|---|---|---|---|---|
| **-4** | 0.0616 | | 112.89% | 0.0400 | 103.62% |
| **-5** | 0.0623 | | 115.28% | 0.0406 | 105.18% |
| **-6** | 0.0395 | | 72.92% | 0.0273 | 70.72% |
| **Control** | 0.0386 | | 100% | | |

These results would indicate that there is melanin synthesis stimulation in the - 4 and -5 dilutions, even when the -6 dilution shows inhibition. This could indicate that if the stimulation existed, it would continue on the second day of treatment, although the values found very much resemble the control values and the -6 dilution shows inhibition as expected. No cytopathic effect was observed.

### EXAMPLE 8

### Melanogenesis inhibition after 3 days of treatment with idebenone

An experiment similar to the one described in the previous example was designed for the purpose of establishing whether ideberone inhibits melanogenesis in cells in culture, but in this case the cells were treated for 5 days and the results were evaluated on the fifth day. The presence or absence of cytopathic effects on the cell cultures was also evaluated by means of using an inverted microscope, evaluating only the -4, -5 and -6 dilutions.

Table XII shows 400 and 475 optical densities (for melanin and dopaquinone, respectively) of the different assayed dilutions and their respective percentages

**Table XII**

| **Dilution** | **OD₄₀₀** | | **Percentage** | **OD₄₇₅** | **Percentage** |
|---|---|---|---|---|---|
| **-4** | 0.0686 | | 77.69% | 0.0513 | 80.66% |
| **-5** | 0.0742 | | 84.03% | 0.0612 | 96.22% |
| **-6** | 0.0801 | | 90.71% | 0.0643 | 101.10% |
| **Control** | 0.0883 | | 100% | 0.0636 | 100% |

These results would indicate that there is dose-dependent melanin synthesis inhibition on the third day of treatment. The amount of dopaquinone in the -6 dilution is similar to the control. No cytopathic effect was observed.

### EXAMPLE 9

### Melanogenesis inhibition mediated by idebenone at high dilutions

An experiment similar to the one described in Example 6 was designed for the purpose of establishing the effect on melanogenesis in cell in culture produced by low concentrations of idebenone, but using -2, -3, -4, -5, -6, -7 and -8 dilutions.

Table XIII shows 400 and 475 optical densities (for melanin and dopaquinone, respectively) of the different assayed dilutions and their respective percentages.

**Table XIII**

| **Dilution** | **OD₄₀₀** | **Percentage** | **OD₄₇₅** | **Percentage** |
|---|---|---|---|---|
| **-2** | 0.01166 | 71.40% | 0.0024 | 14.45% |
| **-3** | 0.01566 | 95.89% | 0.0038 | 22.89% |
| **-4** | 0.01533 | 93.87% | 0.0133 | 80.12% |
| **-5** | 0.01466 | 89.77% | 0.0139 | 83.73% |
| **-6** | 0.00900 | 55.11% | 0.0116 | 69.87% |
| **-7** | 0.01136 | 71.40% | 0.0139 | 83.73% |
| **-8** | 0.01166 | 71.40% | 0.0133 | 80.12% |
| **Control** | 0.01633 | 100% | 0.0166 | 100% |

The results indicate that even when it is very dilu ed, the idebenone compound preserves its ability to inhibit melanogenesis. However, a clear dose-response ratio could not be demonstrated.

### EXAMPLE 10

### Depigmenting capacity of a cream containing 2.5% idebenone in a patient with pregnancy-induced chloasma

The patient was a female patient, 43 years of age, Peruvian nationality, of Inca descent and wheat-colored skin type III, who states that she was pregnant and gave birth 9 years ago and that during the second trimester of pregnancy she exhibited a gravid spot (pregnancy chloasma) in the shape of butterf y wings on both malar regions of the face, a spot which she still has today and increases in pigmentation during the summer months due to sun exposure, and has well defined contrasting edges with the surrounding skin.

Treatment: the patient was instructed to apply a sufficient amount of cream containing 2.5% idebenone, according to Example 1, on the site of the spot every day before going to bed, allowing the cream to remain on the skin overnight and rinsing her face with abundant water and neutral soap the following morning. Application was performed for 20 consecutive days. The patient was clinically examined 7, 14 and 21 days after beginning treatment, comparing the pigmented area with the surrounding skin.

Results: A gradual general decrease of pigmentation of the hyperpigmented skin was observed, becoming noticeable closer to day 2 , although a color similar to the skin without hyperpigmentation was not obtained. The edges of the pigmented macula faded, disappearing, no longer being pronounced and unmistakable, showing degradation from the central area of the chloasma towards the surrounding skin.

Conclusions: the cream containing 2.5% idebenone is effective for attenuating cutaneous pigmentation of gravid chloasma, a condition characterized by localized hyperpigmentation having a likely hormonal etiology. Although treatment was discontinued, the results indicate that the continuation of treatment could lead to the complete disappearance of the hyperpigmentation.

### EXAMPLE 11

### Depigmenting capacity a creaming containing 2.5% idepenone in a patient with post-inflammatory hyperpigmentation

The patient was a female patient, 40 years of age of Argentinean nationality and white skin type II who exhibits significant photoactinic damage and who states she was pregnant 3 times and gave birth 3 times 9, 11 and 14 years ago, and after a hair removal process with wax on the external region of the mouth, has residual hyperpigmentation in the affected area that has lasted for over 3 years, and still persists today, the pigmentation thereof increasing during the summer months due to sun exposure. The hyperpigmentation has well-defined edges contrasting with the surrounding skin.

Treatment: the patient was instructed to apply a sufficient amount of cream containing 2.5% idebenone, according to Example 1, on the site of the spot every day before going to bed, allowing the cream to remain on the skin overnight and rinsing her face with abundant water and neutral soap the following morning. Application was performed for 20 consecutive days. The patient was clinically examined 7, 14 and 21 days after beginning treatment, comparing the pigmented area with the surrounding skin. The patient was instructed to refrain from direct exposure to solar radiation throughout the entire treatment period.

Results: A general decrease of pigmentation of the hyperpigmented skin was observed 7 days after beginning treatment, becoming substantial closer to day 14, and closer to day 21 the edges of the hyperpigmented area were modified, and the size of the macula decreased in an irregular manner. The edges of the pigmented macula faded, disappearing in some areas.

Conclusions: the cream containing 2.5% idebenone is effective for attenuating post-inflammatory cutaneous pigmentation, a condition characterized by localized hyperpigmentation as a result of a dermal inflammatory process. Although treatment was discontinued, the results indicate that the continuation of treatment could lead to the complete disappearance of the hyperpigmentation.

### EXAMPLE 12

### Depigmenting capacity of a cream containing 5% idebenone in a patient with post-medicinal hyperpigmentation

This patient is a female patient, 46 years of age of Argentinean nationality and wheat-colored skin type II, who states that she was pregnant once and gave birth once 8 years ago and who has plaque psoriasis on both elbows. The patient states that after treatment with psoralenes she exhibited hyperpigmented maculae on both elbows on which psoriasis plates still develop, which remit and become exacerbated, always leaving the hyperpigmented base more extensive than the area affected by the psoriasis. The hyperpigmentation became permanent about 5 years ago.

Treatment: the patient was instructed to apply a sufficient amount of cream containing 5% idebenone, according to Example 1, on the site of the spot every day before going to bed, allowing the cream to remain on the skin overnight and rinsing the application site with abundant water and neutral soap the following morning. The application was performed for 15 consecutive days. The patient was clinically examined 7 and 15 days after beginning treatment, comparing the pigmented area with the surrounding skin. The patient was instructed to refra n from direct exposure to solar radiation throughout the entire treatment period. The pigmented area was compared again with the surrounding skin 6 months after treatment was carried out.

Results: A general decrease of pigmentation of the hyperpigmented skin was observed 7 days after beginning treatment, becoming more noticeable closer to day 15, the size and color of the hyperpigmented areas decreasing and the area acquiring the color of the surrounding skin.

Conclusions: the cream containing 5% idebenone is effective for attenuating post-medicinal cutaneous pigmentation.

### EXAMPLE 13

### Shelf-life stability study of a formulation containing 0.3% Idebenone

The stability of two formulations prepared on different dates but with an identical composition was studied. Both were formulated in aqueous cream form (O/W) containing 0.3% idebenone.

The oldest analyzed formulation was packaged ir a white polystyrene jar and was stored on a shelf for a period of 600 days at room temperature and protected from light.

Table XIV shows the quantitative formula of an aqueous cream (O/W) containing 0.3% idebenone. The amounts are for 100 grams.

| **COMPONENTS** | **% BY WEIGHT** |
|---|---|
| Water | 79.7 |
| Non-ionic autoemulsifiable wax | 6.00 |
| Vaseline | 5.00 |
| Glycerin | 5.00 |
| Isopropyl myristate | 2.00 |
| Cetyl alcohol | 1.50 |
| Methylparaben (Nipagin) | 0.20 |
| Propyparaben (Nipasol) | 0.10 |
| Idebenone | 0.3 |

The methodology used for quantifying the active ingredient was UV-Vis spectrophotometry.

A negligible difference was obtained in the assay of the active ingredient for the more recently prepared creams (1 month after their preparation) and the older creams (20 months after their preparation). It is concluded that this cream can be considered stable for at least 600 c ays (20 months).

### EXAMPLE 14

### Shelf-life stability study of a formulation containing 3% ldebenone

The stability of two formulations prepared on different dates but with an identical composition was studied. Both were formulated in aqueous cream form (O/W) containing 3% idebenone.

The oldest ana yzed formulation was packaged in a white polystyrene jar and was stored on a shelf for a period of 600 days at room temperature and protected from light.

Table XIV shows the quantitative formula of an aqueous cream (O/W) containing 3% idebenone. The amounts are for 100 grams

| **COMPONENTS** | **% BY WEIGHT** |
|---|---|
| Water | 79.7 |
| Non-ionic autoemulsifiable wax | 6.00 |
| Vaseline | 5.00 |
| Glycerin | 5.00 |
| Isopropyl myristate | 2.00 |
| Cetyl alcohol | 1.50 |
| Methylparaben(Nipagin) | 0.20 |
| Propyparaben(Nipasol) | 0.10 |
| Idebenone | 3.00 |

The methodology used for quantifying the active ingredient was UV-Vis spectrophotometry.

A negligible difference was obtained in the assay of the active ingredient for the more recently prepared creams (1 month after their preparation) and the older creams (20 months after their preparation). It is concluded that this cream can be considered stable for at least 600 days (20 months).

## Claims

1. A composition comprising idebenone for use in the treatment of hyperpigmentation of skin due to skin disorders, for topical application on the skin.

2. Non-therapeutic use of idebenone in a cosmetic composition for reducing the coloration of the skin in the application site.

3. A composition for use according to claim 1, wherein the skin disorders are selected from psoriasis, rosacea, photodamaged skin, atopic dermatitis, post-medicinal hyperpigmentation, post-inflamatory hyperpigmentation pregnancy-induced chloasma and seborrheic dermatitis.

4. A pharmaceutical and/or dermatological composition comprising an effective amount of idebenone for use in the treatment of hyperpigmentation of skin due to skin disorders for topical application on the skin.

5. A pharmaceutical and/or dermatological composition for use according to claim 4 wherein the skin disorders are selected from psoriasis, rosacea, photodamaged skin, atopic dermatitis, post-medicinal hyperpigmentation, post-inflamatory hyperpigmentation pregnancy-induced chloasma and seborrheic dermatitis.

6. Pharmaceutical and/or dermatological composition for use according to claim 4, wherein the amount of idebenone is comprised between 0.1 % and 10% w/w.

7. Pharmaceutical and/or dermatological composition for use according to claim 4, wherein the amount of idebenone is comprised between 0.3% and 5% w/w.

8. Pharmaceutical and/or dermatological composition for use according to any of claims 4 -7, wherein the composition is in occlusive patch form.

9. Pharmaceutical and/or dermatological composition for use according to any of claim 4 -7, wherein the composition is in cream form.

10. Pharmaceutical and/or dermatological composition for use according to any of claim 4 -7, wherein the composition is in gel form.

11. Pharmaceutical and/or dermatological composition for use according to any of claim 4-7, wherein the composition is in emulsion form.

12. Pharmaceutical and/or dermatological composition for use according to any of claim 4 -7, wherein the composition is in aerosol form.

13. Pharmaceutical and/or dermatological composition for use according to any of claim 4 -7, wherein the idebenone is liposomated, complexed or in a controlled release system.

14. Pharmaceutical and/or dermatological composition for use according to any of claim 4 -7, wherein it comprises oily components selected from autoemulsifiable wax, vaseline, isopropyl myristate and cetyl alcohol and hydrosoluble components selected from glycerin, methylparaben and propylparaben.

15. Pharmaceutical and/or dermatological composition for use according to any of claim 4 -7, wherein it comprises sun screens and/or filters.

16. Pharmaceutical and/or dermatological composition for use according to any of claim 4 -7, wherein it comprises cosmetically and/or pharmaceutically acceptable antioxidizing agents.

## Patentansprüche

1. Zusammensetzung, umfassend Idebenon zur Verwendung in der Behandlung einer Hyperpigmentierung der Haut aufgrund von Hauterkrankungen, zur topischen Anwendung auf der Haut.

2. Nicht therapeutische Verwendung von Idebenon in einer kosmetischen Zusammensetzung zur Verringerung der Färbung der Haut an der Anwendungsstelle.

3. Zusammensetzung zur Verwendung nach Anspruch 1, wobei die Hauterkrankungen ausgewählt sind aus Psoriasis, Rosacea, lichtgeschädigter Haut, atopischer Dermatitis, postmedizinischer Hyperpigmentierung, postinflammatorischer Hyperpigmentierung, durch Schwangerschaft bedingtes Chloasma und seborrhoische Dermatitis.

4. Pharmazeutische und/oder dermatologische Zusammensetzung, umfassend eine wirksame Menge an Idebenon zur Verwendung in der Behandlung einer Hyperpigmentierung der Haut aufgrund von Hauterkrankungen, zur topischen Anwendung auf der Haut.

5. Pharmazeutische und/oder dermatologische Zusammensetzung zur Verwendung nach Anspruch 4, wobei die Hauterkrankungen ausgewählt sind aus Psoriasis, Rosacea, lichtgeschädigter Haut, atopischer Dermatitis, postmedizinischer Hyperpigmentierung, postinflammatorischer Hyperpigmentierung, durch Schwangerschaft bedingtes Chloasma und seborrhoische Dermatitis.

6. Pharmazeutische und/oder dermatologische Zusammensetzung zur Verwendung nach Anspruch 4, wobei die Menge an Idebenon zwischen 0,1% und 10% w/w beträgt.

7. Pharmazeutische und/oder dermatologische Zusammensetzung zur Verwendung nach Anspruch 4, wobei die Menge an Idebenon zwischen 0,3% und 5% w/w beträgt.

8. Pharmazeutische und/oder dermatologische Zusammensetzung zur Verwendung nach einem der Ansprüche 4 bis 7, wobei die Zusammensetzung die Form eines okklusiven Patch aufweist.

9. Pharmazeutische und/oder dermatologische Zusammensetzung zur Verwendung nach einem der Ansprüche 4 bis 7, wobei die Zusammensetzung die Form einer Creme aufweist.

10. Pharmazeutische und/oder dermatologische Zusammensetzung zur Verwendung nach einem der Ansprüche 4 bis 7, wobei die Zusammensetzung die Form eines Gels aufweist.

11. Pharmazeutische und/oder dermatologische Zusammensetzung zur Verwendung nach einem der Ansprüche 4 bis 7, wobei die Zusammensetzung die Form einer Emulsion aufweist.

12. Pharmazeutische und/oder dermatologische Zusammensetzung zur Verwendung nach einem der Ansprüche 4 bis 7, wobei die Zusammensetzung die Form eines Aerosols aufweist.

13. Pharmazeutische und/oder dermatologische Zusammensetzung zur Verwendung nach einem der Ansprüche 4 bis 7, wobei das Idebenon liposomiert, komplexiert oder in einem System mit kontrollierter Freisetzung ist.

14. Pharmazeutische und/oder dermatologische Zusammensetzung zur Verwendung nach einem der Ansprüche 4 bis 7, wobei diese ölige Komponenten, die ausgewählt sind aus selbstemulgierbarem Wachs, Vaseline, Isopropylmyristat und Cetylalkohol, und wasserlösliche Komponenten, die ausgewählt sind aus Glycerin, Methylparaben und Propylparaben, umfasst.

15. Pharmazeutische und/oder dermatologische Zusammensetzung zur Verwendung nach einem der Ansprüche 4 bis 7, wobei diese Sonnenschutz- und/oder -filtermittel umfasst.

16. Pharmazeutische und/oder dermatologische Zusammensetzung zur Verwendung nach einem der Ansprüche 4 bis 7, wobei diese kosmetisch und/oder pharmazeutisch annehmbare Antioxidationsmittel umfasst.

## Revendications

1. Composition comprenant de l'idébénone utilisable dans le traitement d'une hyperpigmentation cutanée due à des affections cutanées, pour une application topique sur la peau.

2. Utilisation non thérapeutique de l'idébénone dans une composition cosmétique pour réduire la coloration de la peau sur le site d'application.

3. Composition utilisable selon la revendication 1, dans laquelle les affections cutanées sont choisies parmi le psoriasis, la rosacée, le photovieillissement de la peau, l'eczéma constitutionnel, l'hyperpigmentation d'origine médicamenteuse, l'hyperpigmentation post-inflammatoire, le masque de grossesse et la dermite séborrhéique.

4. Composition pharmaceutique et/ou dermatologique comprenant une quantité efficace d'idébénone utilisable dans le traitement d'une hyperpigmentation cutanée due à des affections cutanées, pour une application topique sur la peau.

5. Composition pharmaceutique et/ou dermatologique utilisable selon la revendication 4, dans laquelle les affections cutanées sont choisies parmi le psoriasis, la rosacée, le photovieillissement de la peau, l'eczéma constitutionnel, l'hyperpigmentation d'origine médicamenteuse, l'hyperpigmentation post-inflammatoire, le masque de grossesse et la dermite séborrhéique.

6. Composition pharmaceutique et/ou dermatologique utilisable selon la revendication 4, dans laquelle la quantité d'idébénone est comprise entre 0,1 et 10 % p/p.

7. Composition pharmaceutique et/ou dermatologique utilisable selon la revendication 4, dans laquelle la quantité d'idébénone est comprise entre 0,3 et 5 % p/p.

8. Composition pharmaceutique et/ou dermatologique utilisable selon l'une quelconque des revendications 4 à 7, dans laquelle la composition est sous la forme d'un patch occlusif.

9. Composition pharmaceutique et/ou dermatologique utilisable selon l'une quelconque des revendications 4 à 7, dans laquelle la composition est sous la forme d'une crème.

10. Composition pharmaceutique et/ou dermatologique utilisable selon l'une quelconque des revendications 4 à 7, dans laquelle la composition est sous la forme d'un gel.

11. Composition pharmaceutique et/ou dermatologique utilisable selon l'une quelconque des revendications 4 à 7, dans laquelle la composition est sous la forme d'une émulsion.

12. Composition pharmaceutique et/ou dermatologique utilisable selon l'une quelconque des revendications 4 à 7, dans laquelle la composition est sous la forme d'un aérosol.

13. Composition pharmaceutique et/ou dermatologique utilisable selon l'une quelconque des revendications 4 à 7, dans laquelle l'idébénone est contenue dans des liposomes, complexée ou contenue dans un système à libération contrôlée.

14. Composition pharmaceutique et/ou dermatologique utilisable selon l'une quelconque des revendications 4 à 7 comprenant des composants huileux choisis parmi la cire auto-émulsifiable, la vaseline, le myristate d'isopropyle et l'alcool cétylique et des composants hydrosolubles choisis parmi le glycérol, le p-hydroxybenzoate de méthyle et le p-hydroxybenzoate de propyle.

15. Composition pharmaceutique et/ou dermatologique utilisable selon l'une quelconque des revendications 4 à 7 comprenant des écrans et/ou des filtres solaires.

16. Composition pharmaceutique et/ou dermatologique utilisable selon l'une quelconque des revendications 4 à 7 comprenant des agents antioxydants acceptables sur le plan cosmétique et/ou pharmaceutique.
